# EUROPEAN PATENT SPECIFICATION

(11) **EP 1 326 993 B1**
(45) Date of publication and mention of the grant of the patent: **23.05.2007**
(21) Application number: 01983974.5
(22) Date of filing: 17.10.2001
(51) Int. Cl.: C12P 7/42

(54) **PRODUCTION OF ALPHA-HYDROXY-CARBOXYLIC ACIDS USING A COUPLED ENZYME SYSTEM**
HERSTELLUNG VON ALPHA -HYDROXY-CARBOXY SÄURE MIT HILFE VON EINEM GEKOPPELTEN ENZYM SYSTEM
PRODUCTION D'ACIDES ALPHA-HYDROXY-CARBOXYLIQUES À L'AIDE D'UN SYSTÈME D'ENZYMES COUPLÉS

(30) Priority: 17.10.2000 US 241177 P
(43) Date of publication of application: 16.07.2003
(73) Proprietor: Excelsyn Molecular Development Limited, Holwell Flintshire Wales CH8 9DN (GB)
(72) Inventor: SENKPEIL, Richard, F., Lisle, IL 60532 (US); PANTALEONE, David, P., Buffalo Grove, IL 60089 (US); TAYLOR, Paul, P., Arlington Heights, IL 60004 (US)
(74) Representative: Pidgeon, Robert John
(86) International application number: PCT/US2001/032243
(87) International publication number: WO 2002/033110

(56) References cited:
- WO-A-92/12249
- US-A- 5 686 275
- US-A- 6 033 882
- MASSAD G ET AL: "PROTEUS MIRABILIS AMINO ACID DEAMINASE: CLONING, NUCLEOTIDE SEQUENCE, AND CHARACTERIZATION OF AAD" JOURNAL OF BACTERIOLOGY, WASHINGTON, DC, US, vol. 177, no. 20, October 1995 (1995-10), pages 5878-5883, XP001055638 ISSN: 0021-9193 cited in the application
- HANSON R.L. ET AL: 'Transformation of N[epsilon]-CBZ-L-lysine to CBZ-L-oxylysine using L-amino acid oxidase from Providencia alcalifaciens and L-2-hydroxy-isocaproate dehydrogenase from Lactobacillus confusus' APPLIED MICROBIOLOGY AND BIOTECHNOLOGY vol. 37, no. 5, August 1992, GERMANY, pages 599 - 603, XP009054262

<110> PCBU SERVICES, INC. et al
<120> PRODUCTION OF ALPHA-HYDROXY-CARBOXYLIC ACIDS USING A COUPLED ENZYME SYSTEM
<130> 29479/501A-NSC
<140> PCT/US01/32243
   <141> 2001-10-17
<150> US 60/241,177
   <151> 2000-10-17
<160> 2
<170> PatentIn version 3.1
<210> 1
   <211> 62
   <212> DNA
   <213> Artificial sequence
<220>
   <223> Primer
<400> 1
   tttggatcca aaatgaacat ctctcgtcgt aaactgctgt taggtgttgg tgctgcgggc 60
   gt 62
<210> 2
   <211> 59
   <212> DNA
   <213> Artificial sequence
<220>
   <223> Primer
<400> 2
   agctttgtcg acgggccctt agtgatggtg atggtgatgc ttcttaaaac gatccaaac 59

## Description

### FIELD OF THE INVENTION

This invention relates to an economical, expedient, and versatile method of synthesizing either the R- or S-isomer of an α-hydroxy-carboxylic acid with very high enantiomeric purities at the α-hydroxy center from an α-amino-carboxylic acid. The transformation is catalyzed by a coupled enzyme system of an amino acid deaminase (AAD, also referred to as amino acid oxidase, AAO), a lactate dehydrogenase (LDH), an electron donor, and an enzyme/substrate system for recycling the electron donor. This invention also relates to the use of α-amino-carboxylic acids, hydrates, and salts thereof and a coupled enzyme system in the production of α-hydroxy-carboxylic acids, hydrates, and salts thereof.

### BACKGROUND OF THE INVENTION AND THE PRIOR ART

The synthesis of chiral α-hydroxy (also sometimes referred to as 2-hydroxy) carboxylic esters, acids, and their salts is of considerable importance, since these compounds are versatile synthetic intermediates which are often converted into a variety of useful chiral compounds, especially pharmaceuticals. The chirality of the α-hydroxy-carboxylic esters, acids and their salts maybe parlayed into other chiral functionalities either with retention of the stereochemistry at the α-position, for example, epoxides, alkyl esters, hydrazinyl esters, α-N-alkoxyamino esters and α-amino esters, or with inversion of the stereochemistry *via* reactions involving nucleophilic substitution at the α-position, for example reaction of the corresponding α-triflate esters with a suitable nucleophile.

The availability of chiral α-hydroxy-carboxylic esters, acids, hydrates, and salts thereof possessing additional prochiral functional groups in the molecule offers further potential for the synthesis of compounds containing additional chiral centers. There are numerous examples where the hydroxyl group at the α-position provides an internal control element, facilitating stereoselective transformations of a prochiral functional group. For example, epoxidation of β-γ-unsaturated compounds gives access to Sharpless-type intermediates, dehydroxylation of β-γ-unsaturated compounds produces polyols which have utility in carbohydrate synthesis, and diastereoselective reduction of 4-oxo compounds provides either syn- or anti-1,3-diols, depending on the choice of conditions. Since α-hydroxy-carboxylic esters, acids, hydrates, and salts thereof are such valuable synthetic entities, much effort has been expended in the development of methods for their preparation in chiral form and examples of both traditional chemical and more recent enzymatic methods are described below.

There are very few examples of traditional chemical methods that directly produce α-hydroxy-carboxylic acids or their salts from α-amino-carboxylic acids or their salts since the key transformation generally involves the use of water-sensitive reagents and low temperatures on extremely polar compounds at a relatively unreactive and stereochemically sensitive center. Deamination ofD-amino-carboxylic acids by nitration and hydrolysis (Winitz M., et al. *J*. *Am. Chem. Soc.* 1956, **78,** 2423) can provide access to α-hydroxy-carboxylic acids of the L-configuration, but requires the use of expensive starting materials of limited availability. The use of the more readily available and inexpensive L-amino-carboxylic acids as starting materials (Kunz H., et al. *Tet. Lett.* 1987, **28**, 1873) requires several steps, reaction times of several weeks, has the potential to form numerous side products, and results in only moderate yields of only the D-hydroxy-carboxylic acid derivatives.

There are many examples of methods of synthesizing α-hydroxy-carboxylic acids from α-keto carboxylic acids or esters, however, these methods are also limited in their general utility, especially since the starting materials lack an asymmetric center, are also relatively expensive and of limited availability. With the exception of enone reductions, product chirality arises either from the stoichiometric use of a chiral auxiliary (substrate control) requiring several synthetic steps or a bulky and costly chiral reductant (reagent control). For example, asymmetric reduction of α-keto esters using chiral 9-borabicyclo[3.3.1]nonane (9-BBN) derivatives (Brown H.C. et. al. *J. Org. Chem.* 1986 51, 3396) requires stoichiometric quantities of these complex reducing agents. Hydroxylation of chiral oxazolidone enolates with oxazolidone oxidants (Evans, D.A. et al. *J*. *Am. Chem Soc.,* 1985, **102**, 4346) requires the chromatographic resolution of the α-hydroxy imide intermediates be undertaken prior to an additional solvolysis step and results in poor yields when hindered α-keto carboxylic acids are used. Carboxylation of chiral α-alkoxycarbanions (Chan C.M. et. al. *Tet. Lett.* 1990, **31**, 1985) requires a stoichiometric quantity of the expensive reducing agents (R)- or (S)-BINAL-H and requires the disposal of hazardous tin residues after the transmetallation stage. In contrast to the above methods, enantioselective reduction of enones can be acheived using only a catalytic amount of a chiral oxazaborolidine, (Coney, G.J. et. al. *Tet. Lett.,* 1990, **31**, 611), however a sequence of four chemical conversions are required in order to transform the initially formed chiral alcohol to a α-hydroxy ester with obvious cost and yield implications.

Enzymatic methods that produce chiral α-hydroxy-carboxylic acids also rely on α-keto carboxylic esters or acids as precursors and are catalyzed by purified or isolated reductases (e.g. Ghisalba, O. et al. U.S. Patent No. 5,098,841; Wandrey, C. et al. U.S. Patent No. 4,326,031; and Casey, G. et al. U.S. Patent No. 5,686,275). There are no published processes that utilize α-amino-carboxylic acids or their salts as starting materials. These enzymatic reduction methods generally require the addition of an electron donor, typically a reduced pyridine nucleotide or flavine nucleotide, and an efficient system its regeneration, i.e. the addition of a second enzyme and its substrate. As with enone reductions, these methods also have the advantage of requiring only a catalytic amount of the chiral reducing agent (in these cases, the enzyme and its cofactor) and have the potential to produce chiral α-hydroxy-carboxylic acids from prochiral substrates.

If these biocatalytic methods are to be practical, they must be cost effective over other methods and the resulting product must be produced with a high isomeric purity and chemical yield. These factors are generally a function of the enzyme systems durability, specificity, and rate of substrate turnover. The origin of the enzymes used is of crucial importance to the properties and efficacy of these enzymatic reactions. Even enzymes of the same type differ with regard to both structural factors such as reaction specificity, substrate specificity, stereospecificity, and kinetic factors such as the Michaelis-Menten constant (K*_{M}*) and the inhibition constant (K*_{I}*) if they have been isolated from different sources, for example, different microorganisms. For example, D-lactate dehydrogenase from *Lactobacillus confusus* converts pyruvate, α-ketobutyrate, and phenylpyruvate, but does not reduce α-ketovalerate, α-ketocaproate and α-keto-β-methylvalerate, while D-lactate dehydrogenase from *Staphylococcus epidermidis (S. epidennidis)* converts pyruvate, phenylpyruvate, α-ketobutyrate but not α-keto-β-rnethylvalerate or 2-oxoadipate. The behavior of individual enzyme/substrate systems must therefore, be tested for each case and cannot be predicted by generalization, although some references, point to that conclusion (e.g. European Patent 0 024 547). While there are numerous descriptions reported in the literature of these enzymatic conversions, some of which are cited below, they have customarily resulted in low yields and their general utility remains to be demonstrated.

An (R)-oxynitrilase catalyses the synthesis of chiral cyanohydrins which are subsequently hydrolyzed (Zeigler, T., et al., *Synthesis* 1990, 575) to give access to (R)-α-hydroxy-carboxylic acids. A highly toxic water-free preparation of hydrogen cyanide is required for the enzymatic reaction, which gives variable enantioselectivities as low as 74% ee of only the R-isomer. The use of (S)-oxynitrilases is generally described in Topics in Current Chemistry, Vol. 200, Springer Verlag, Berlin (1999), 193-226.

Examples of enzymatic kinetic resolution include those catalysed by *Pseudomonas fluorescens* lipase giving chiral α-hydroxy esters (Kalaritis P., et al., *J*. *Org. Chem.* 1990*,* **55**, 812) and those catalyzed by glycolate oxidase and D-lactate dehydrogenase (Adam W. et al., Tetrahedron Asymmetry 1998,9,351) giving chiral α-hydroxy acids. These methods are inherently flawed since yields of a particular enantiomer are limited to a maximum of 50% in resolutions of unsymmetrical substrates. In practice, yields are further reduced since the percent conversion has to be carefully controlled in order to achieve high optical purities.

A number of workers have investigated the reduction of α-oxo carboxylic acids catalyzed by S-lactate dehydrogenases (S-LDH or L-LDH) isolated from a variety of sources, including those obtained from mammalian tissues (e.g. rabbit muscle and porcine and beef heart, Kim, M.-J. et al. *J*. *Am. Chem. Soc.* 1988, **110**, 2959) and bacteria (e.g. *Bacillus stearothermophilus,* Luyten, M. A. et. al. *J*. *Am. Chem. Soc.* 1989, **111,** 6800). Numerous α-oxo carboxylic acids have been described in the literature as exhibiting measurable activity against S-LDH as determined by following the course of NADH oxidation, but only in some cases have preparative-scale experiments been performed, the product α-hydroxy-carboxylic acids fully characterized, and the enantioselectivity of the reduction established. The compounds investigated as substrates also tend to anticipate the possible useful boundaries ofreduction with α-oxo-carboxylic acid dehydrogenases. The general utility of these reactions, therefore, is subject to interpretation. When fully characterized, these methods, for the most part, provide (S)-α-hydroxy-carboxylic acids in good chemical yields and high optical purities. The reactivity of some substrates is limited, however, and may require the use of allosteric activators, such as fructose 1.6-bisphosphate (FBP), and can still take over a week to proceed to completion. This compares to typical reaction times of 1-3 days.

In order to obtain α-hydroxy-carboxylic acids with the (R)-absolute configuration, reduction of α-oxo-carboxylic acids catalyzed byR-LDH or D-LDH has also been investigated. This enzyme can be isolated on a large scale (Hummel, W. et. al. *Eur*. *J. Appl. Microbiol. Biotechnol.* 1983,**18**, 75). Recent studies (Simon, et al., Appl. Biochem. Biotechnol. 1989, **22**, 169 and Kim M. J. et. al. *Chem. Soc. Chem. Commun.* 1991, 326) have focused on R-LDH from *Leuconostoc mesenteroides* (LM-R-LDH) and *Staphylococcus epidermidis* (SE-R-LDH). Only a limited number of α-oxo carboxylic acid substrates have been shown to exhibit measurable activity and been reduced on a preparative scale.

As mentioned previously, when using purified or isolated reductases an efficient system for enzymatic regeneration of the coenzyme, i.e. a second enzyme and its substrate is required. For these reactions, a catalytic amount of NADH is typically employed in conjunction with a regenerating system, requiring a second enzyme, usually formate dehydrogenase (FDH), which utilizes NAD⁺ in the oxidation of formate ion to carbon dioxide (Shaked Z. et. al. *J*. *Am. Chem. Soc.* 1980, **102**, 7105). Yamazaki & Maeda describe a batch and continuous process *(Agricol. Biol. Chem.* 1986, **50**, 2621 and 3213, respectively) for the synthesis ofR-(-)-mandelic acid from benzoyl formate with the aid of NADH and the benzoyl formate dehydrogenase from *Streptococcus faecalis.* European Patent 0 024 547 also describes a process for the continuous enzymatic conversion of water-soluble α-ketocarboxylic acids into the corresponding α-hydroxycarboxylic acids in an enzyme membrane reactor. The conversion is carried out in the presence of NAD(H) of which the molecular weight has been increased by bonding to polyethylene glycol and in the presence of a lactate dehydrogenase with simultaneous NADH regeneration by formate dehydrogenase and formate.

The production of chiral α-hydroxy-carboxylic acids by stereospecific microbiological conversion has also been utilized, although this also has been limited to reductions using α-keto carboxylic acids as precursors. Either intact microorganisms, for example fungi (e.g. *Mucor, Geotrichum, Saccharomyces, Candida)* or bacteria (e.g. *Proteus, Pseudomonas)* or their extracts have been used as the biocatalysts. The reduction of the substrate in these cases is also effected by a substrate-specific dehydrogenase. Electron donors are also required for this process and can be, for example, carbohydrates (e.g. glucose), formate, ethanol, hydrogen, or the cathode of an electrochemical cell, but are also generally provided by a coenzyme, e.g. by pyridine nucleotides such as reduced nicotinamide adenine dinucleotide (NADH) and reduced nicotinamide adenine dinucleotide phosphate (NAPDH) or by reduced flavin nucleotides such as reduced flavine mononucleotide (FMNH) and reduced flavine adenine dinucleotide (FADH). The reduced coenzymes are in turn usually produced in a series of enzyme-catalyzed steps in which competing electron acceptors are formed or by electron transfer by natural or synthetic mediators (e.g. ferredoxin, viologens), however, some reductases are known that are able to accept electrons directly from the mediators. All of these systems are still limited, however by the cost and availability of the starting α-keto carboxylic acids.

Since the cost and chemical availability of the starting α-keto carboxylic acids can be prohibitive, a promising method for the production of α-keto carboxylic acids is the utilization of amino acid deaminases or oxidases (e.g. L-amino acid: oxygen oxidoreductase; EC 1.4.3.2) which transform readily available and inexpensive L-amino-carboxylic acids to the corresponding α-keto carboxylic acids (Massad, G. et al. *J. of Bact.,* 1995*,* **177***,* No. 20, 5878) and the reverse (Pantaleone, D. et al. *Journal of Molecular Catalysis B: Enzymatic,* 2001, 11: 795; Galkin, Andrey, et al. *Appl. Environ. Microbiol.,* 1997, **63,** No. 12, 4651). Amino acid oxidases have been studied in and isolated from a number of sources (as shown in Table 1) although typically, they are not commercially available.

**Table 1. Sources of L-Amino Acid Oxidases**

| **Organism** | **Type** | **Reference** |
|---|---|---|
| *Proteus rettgeri* | Bacterium | Chen, S. S. et. al., *Arch. Biochem. Biophys. 146:54-63,* (1971); Duerre, J. A. et. al., *J*. *Bacteriol. 121: 656-63,* (1975). |
| *Cellulomonas cellulans* AM8 | Bacterium | Braun, M. et. al., *Appl. Microbiol. Biotechnol. 37:594-598,* (1992)*.* |
| *Bacillus carotarum* 2Pfa | Gram positive bacterium | Brearley, G. M. et al., *Arch. Microbiol. 161:409-413,* (1994); Brearley, G. M. et al., *Appl. Microbiol. Biotechnol. 41:670-676,* (1994). |
| *Proteus vulgaris* | Bacterium | Stumpf, P. K. et al., *J. Biol. Chem. 153:387-399,* (1944); Takahashi, E. et. al., *Appl. Microbiol. Biotechnol. 47:173-179,* (1997); Takahashi, E. et. al., *Biosci. Biotechnol. Biochem. 63:2244-2247*, (1999). |
| *Proteus mirabilis* | Bacterium | Laboure, A.-M. et. al., *J*. *Bacteriol. 137:161-168*, (1979); Massad, G. et. al., *J. Bacteriol. 177: 5878-83,* (1995); Pelmont, J. et. al., *Biochimie. 54:1359-74,* (1972); Pelmont, J. et. al., *Biochimie. 60:817-821,* (1978); Cioaca C. et. al. *Arch. Roum. Pathol. Exp. Microbiol. 33: 211,* (1974). |
| *Morganella morganii* | Gram negative bacterium | Bouvrette, P. et. al., *Appl. Biochem. Biotechnol. 48:61-74,* (1994); Gamati, S. et al., *Bioseparation. 2:147-154*, (1991). |
| *Pseudomonas sp.* P-501 | Bacterium | Koyama, H., J. *Biochem. 93:1313-1319,* (1983); Koyama, H., *J. Biochem. 92:1235-1240,* (1982). |
| *Providencia sp.* PCM 1298 | Bacterium | Szwajcer, E. et al., *Enzyme Microb. Technol. 4:409-13,* (1982). |
| *Providencia alcalifaciens* | Bacterium | Hanson, R. L. et. al., *Appl. Microbiol. Biotechnol. 37:599-603,* (1992). |
| *Corynebacterium* | Bacterium | Coudert, M. et. al., *Arch. Microbiol. 102:151-153,* (1975). |
| *Calloselasma rhodostoma* | Malayan Pit Viper | Ponnudurai, G. et. al., *Arch. Biochem. Biophys. 313:373-378,* (1994). |
| *Crotalus terrificus terrificus* | Snake venom | Chen, S. S. et. al., *Arch. Biochem. Biophys. 146.-54-63,* (1971). |
| *Crotalus adamanteus* | Eastern Diamond-back rattlesnack | Wellner, D., et. al., J. *Biol. Chem. 235:2013-2018,* (1960). |
| *Crotalus atrox* | Western Diamondback rattlesnack | Torii, S. et. al., *J. Biol. Chem. 272:9539-9542.,* (1997); Torii, S. et. al., *Biochemistry. 39:3197-3205,* (2000). |
| *Agkistrodon piscivorus piscivorus* | Cottonmouth moccasin | Singer, T. P. et. al., *Arch. Biochem. 29:190-209,* (1950). |
| *Trimeresurus mucrosquamatus* | Taiwan habu snake | Ueda, M., et. al., *Toxicon. 26: 695-706,* (1988). |
| *Ophiophagus hannah* | King cobra | Tan, N.-H. et. al., *Int. J. Biochem. 23:323-327,* (1991); Tan, N. H. et. al., *Biochem. Int. 19:937-944,* (1989). |
| *Naja naja kaouthia* | Monocellate cobra | Tan, N.-H. et. al., *Int. J. Biochem. 24:967-973,* (1992)*.* |
| *Synechococcus* PCC 7942 | Cyano-bacterium | Bockholt, R. et. al., *Biochim. Biophys. Acta. 1307:111-121,* (1996); Engels, D. H. et. al., Z. *Naturforsch. 47c:859-866,* (1992). |
| *Synechococcus* PCC 6301 | Cyano-bacterium | Bockholt, R. et. al., *Biochim. Biophys. Acta. 1264:289-293,* (1995). |
| *Anacystis nidulans* | Cyano-bacterium | Pistorius, E. K. et. al., *Biochim. Biophys. Acta. 611:227-240,* (1980)*.* |
| *Amphiora crassissima* | Red alga | Ito, K. et. al., *Yendo. Hydrobiologia. 151*/*152:363-369,* (1987). |
| *Gymnogongrus flabelliformis* | Red alga | Fujisawa, S., et.al., *Bull. Jpn. Soc. Sci. Fisheries 48:97-103,* (1982). |
| *Chlamydomonas reinhardtii* | Green alga | Munoz-Blanco, J. et.al., *Planta. 182:194-198,* (1990); Piedras, P., et. *al., Planta. 188:13-18,* (1992); Vallon, O. et. al., *Eur. J. Biochem. 215:351-360,*(1993)*.* |
| *Pleurochrysis* | Phytoplankton | Palenik, B. et. al., *Limnol. Oceanogr. 35:260-269,* (1990); Palenik, B. et. al., *Mar. Ecol. Prog. Ser. 59:159-201,* (1990). |
| | Rat kidney mitochondria | Nakano, M. et. al., *J. Biol. Chem. 241:2075-2083,* (1966). |
| *Neisseria meningitidis* | | Yu, E. K. C. et. al., *J. Bacteriol. 145:280-287,* (1981). |
| *Trichoderma viride* Y244-2 | Fungus | Kusakabe, H. et. al., *J. Biol. Chem. 255:976-981,* (1980). |
| *Neurospora crassa* | Fungus | Niedermann, D. M. et. al., *J*. *Biol. Chem., 265:17246-17251;* Thayer, P. S. et. al., *J. Biol. Chem. 192: 755-767,* (1951). |

The activity of amino acid deaminases, therefore, has mostly been studied with extracts or whole cells. As with lactate dehydrogenases, only a limited number of compounds have been demonstrated to have measurable activity as substrates of amino acid deaminases from various sources and they tend to anticipate the possible boundaries of oxidation with amino acid deaminases (e.g. only naturally occuring α-amino-carboxylic acids have been described in the literature). The product α-keto carboxylic acids are rarely fully characterized, instead, usually inferred by following the course or the reaction by observing the formation of colored iron complexes. The reactions are widely variable with regard to substrate affinity and specificity, for example the affinity and specificity of amino acid oxidases of the type found in bacteria differs from the flavin amino acid oxidases of the type found in animal cells and snake venom. In addition, they have yet to be performed on a preparative scale. The success of these reactions, therefore, is also subject to interpretation.

The direct formation of the homochiral α-hydroxy-carboxylic acids could be performed using inexpensive and readily available L-α-amino-carboxylic acids or their salts as the raw materials if these enzyme systems could be coupled, the substrate specificity for each enzyme was sufficiently broad, and the turnover rate for each enzyme sufficiently large for the process to be of practical use. As mention previously, L-α-aimno-carboxylic acids and their salts have not been described as a substrates in the prior art relating to the enzymatic production of α-hydroxy-carboxylic acids or their salts and accordingly, have not been investigated with regard to their stability and behaviour in such an enzymatic biotransformation.

The present invention discloses an efficient process for the generation of enantiomerically pure D- and L- α-hydroxy-carboxylic acids, hydrates, and salts thereof by the enantioselective enzymatic oxidation and reduction of α-amino-carboxylic acids, hydrates, and salts thereof using a coupled enzyme system of an amino acid deaminase and either L-lactate dehydrogenase or D-lactate dehydrogenase with NADH as a cofactor. A third enzyme system makes use of the oxidation of formic acid with the formate dehydrogenase to regenerate the cofactor.

### SUMMARY OF THE INVENTION

One aspect of the present invention is to provide a process that allows for the use of inexpensive and readily available α-amino-carboxylic acids or salts thereof as the raw materials for the generation of chiral α-hydroxy-carboxylic acids or salts thereof. The process is characterized in that the amino-carboxylic acids, hydrates, and salts thereof are deaminated to their corresponding α-keto carboxylic acids or salts thereof with a L-amino acid deaminase and subsequently reduced to the target α-hydroxy-carboxylic acids or salts thereof using either L-lactate dehydrogenase from bovine heart, rabbit muscle, porcine heart, or porcine muscle or D-lactate dehydrogenase from *Staphylococcus epidermidis, Leuconostoc mesenteroides, Lactobacillus leichmannii, orBacillus stearothermophilus* with NAD(H) as a cofactor/electron donor. A third enzyme system makes use of the oxidation of formic acid with the formate dehydrogenase to regenerate_the cofactor/electron donor.

Thus, an aspect of the present invention provides a method for the production by a batch process of a chiral α-hydroxycarboxylic acid, hydrate, or salt thereof comprising oxidising an α-amino-carboxylic acid, hydrate, or salt thereof (the substrate) using an L-amino acid deaminase or oxidase; and subsequently reducing the product thereof using a dehydrogenase; wherein the concentration of the substrate is between 0.1 and 2 mol/litre. Preferably the dehydrogenase is an L-lactate dehydrogenase (EC# 1.1.1.28) or a D-lactate dehydrogenase (EC# 1.1.1.27). The source of L- or D-lactate dehydrogenase may suitably be selected from bovine heart, rabbit muscle, porcine heart, porcine muscle, *Staphylococcus epidermis, Leuconostoc mesenteroides, Lactobacillus leichmannii, or Bacillus stearothermophilus.*

The chiral α-hydroxycarboxylic acid may, for example be a sodium salt or a potassium salt. It may be a substituted or unsubstituted (2R) - or (2S)-2-hydroxyalkonic acid. Preferably the chiral α-hydroxycarboxylic acid is selected from the group consisting of (2R)- or (2S)- 2-hydroxybutanoic acid, (2R)- or (2S)- 2-hydroxypentanoic acid, (2R)-or (2S)-2-hydroxyhexanoic acid, (2R)- or (2S)- 2-hydroxy-3-methylpentanoic acid, (2R)- or (2S)- 2-hydroxy-4-pentanoic acid, (2R)- or (2S)- 2,3-dihydroxypropanoic acid, (2R)- or (2S)- 2,4- dihydroxypropanoic acid, (2R)- or (2S)- 3-chloro-2-hydroxypropanoic acid, (2R)- or (2S)- 2-hydroxy-3-mercaptopropanoic acid, (2R)- or (2S)- 2-hydroxy-4-thiomethylbutanoic acid, (2R)- or (2S)- phenyllactic acid, (2R)- or (2S)- p-hydroxyphenyllactic acid, (2R)- or (2S)-2-hydroxy-4-phenylbutanoic acid, (2R)- or (2S)- 2-hydroxy-3-indolylpropanoic acid, (2R)- or (2S)- 3-methyl-2-hydroxybutanoic acid, (2R)- or (2S)- 4-methyl-2-hydroxypentanoic acid, (2R)- or (2S)-4,4-dimethyl-2-hydroxypentanoic acid, (2R)- or (2S)- 3-hydroxy-2-hydroxypropionic acid, (2R)- or (2S)- 2-hydroxy-4-phenylbutanoic acid, (2R)- or (2S)- 3-(2-pyridyl)-2-hydroxypropionic acid, (2R)- or (2S)- 3-(3-pyridyl)-2-hydroxypropionic acid, (2R)- or 2S)- 3-(4-pyridyl)-2-hydroxypropionic acid, (2R)- or (2S)- 3-(1-naphthyl)-2-hydroxypropionic acid, (2R)- or (2S)- 3-(2-naphthyl)-2-hydroxypropionic acid, (2R)-or (2S)- p-hydroxyphenyl-2-hydroxypropionic acid, (2R)- or (2S)- *p*-F-phenyl-2-hydroxypropionic acid, (2R)- or (2S)- α-hydroxyglutaric acid, (2R)- or (2S)- 2-hydroxyadipic acid, (2R)- or (2S)- 2-hydroxy-5-ureidovaleric acid, (2R)- or (2S)- 2-hydroxy-6-ureidonohexanoic acid, (2R)- or (2S)- mercapto-2-hydroxypropionic acid, (2R)- or (2S)- indole-2-hydroxypropionic acid, and a combination thereof.

In the method of the present invention, the oxidising step is preferably carried out with whole cells.

Suitably the method is carried out in a reaction vessel or fermentor:
a) that is equipped with any oxygen sparge;
b) that contains a reaction mixture consisting of a solution of *E.coli,* that was cloned with the gene for L-amino acid deaminase (oxidizing enzyme) and an α-amino-carboxylic acid;
c) to which are fed an aqueous solution of a formate ion (cobustrate), mercaptoethanol, dithiothreitol (antioxidants), Tris-Cl (buffer), nicotinamide adenine dinucleotide (electron donor), D-lactate dehydrogenase (reducing enzyme) and fomrate dehydrogenase (enzyme/substrate system for regenerating/recycling the electron donor); and
d) in which the compound formed is isolated by ion exchange or solvent extraction.

Another aspect of the present invention relates to the use of α-amino-carboxylic acids, hydrates, and salts thereof and a coupled enzyme system in the production of α-hydroxy-carboxylic acids, hydrates and salts thereof.

Yet another aspect of the present invention relates to the use of a process that generates α-hydroxy-carboxylic acids, hydrates, and salts thereof from α-amino-carboxylic acids, hydrates, and salts thereof.

In particular, the present invention provides the use of α-amino-carboxylic acids, hydrates, and salts thereof as substrates for a coupled enzyme system for the production by a batch process of α-hydroxy-carboxylic acids, hydrates, and salt thereof, wherein the substrate concentration is between 0.1 and 2 mol/litre.

### DETAILED DESCRIPTION OF THE PREFERRED EMBODIMENTS OF THE INVENTION

The present invention provides a process that allows for the use of inexpensive and readily available L-α-amino-carboxylic acids, hydrates, and salts thereof as the raw material for the generation of both L- and D-α-hydroxy-carboxylic acids or salts thereof. The substrate amino-carboxylic acids, hydrates, and salts thereof are deaminated to their corresponding α-keto carboxylic acids, hydrates, and salts thereof with the enzyme L-amino acid deaminase and the resulting product subsequently reduced to the target α-hydroxy-carboxylic acids, hydrates, and salts thereof using either the enzyme L-lactate dehydrogenase or D-lactate dehydrogenase in the presence of an electron donor. A third enzyme/substrate system makes use of the oxidation of formic acid with the formate dehydrogenase to regenerate the electron donor/cofactor. The process of this invention permits α-aminocarboxylic acids, hydrates, and salts thereof to be converted with high yields into the corresponding α-hydroxycarboxylic acids, hydrates, and salts thereof and is therefore a useful and cost effective method for the production of these α-hydroxy-carboxylic acids and their salts.

A variety of amino acid oxidases can be used as long as they can process the conversion of α-amino-carboxylic acids, hydrates, and salts thereof to α-keto carboxylic acids, hydrates, and salts thereof. Examples of enzymes usable in the process of the invention are shown in Table 1 and include L-amino acid deaminase, (i.e. L-amino acid oxidase) phenylalanine oxidase, phenylalanine dehydrogenase, and combinations thereof. The use of L-amino acid deaminase cloned in *E*. *coli* is preferred. L-amino acid deaminases from other sources can be used, for example from hog kidney, snake venom, *Proteus mirabilis, Proteus rettgeri, Providencia alcalifaciens, Morganella morganii,* but *E*. *coli* carrying a multi-copy clone of the L-AAD gene has the advantage of much higher specific activity.

A variety of α-ketoacid dehydrogenases can be used as long as they can process the conversion of α-keto carboxylic acids, hydrates, and salts thereof to α-hydroxy-carboxylic acids, hydrates, and salts thereof. Examples of reductases usable in the process of the invention include L or D-Lactate dehydrogenase, L or D-mandalate dehydrogenase, L or D- hydroxyisocaproate dehydrogenase, L or D-benzoylformate dehydrogenase, and combinations thereof. The use of either L-Lactate dehydrogenase and D-lactate dehydrogenase is preferred. Lactate dehydrogenases from other sources can also be used, for example from *Bacillus stearothermophilus,* but compared with lactate dehydrogenases from other microorganisms, the D-LDH from *Staphylococcus epidermidis, Leuconostoc mesonteroides,* or *Lactobacillus leichmannii* and L-LDH from bovine heart are distinguished by a high specific activity (units/mg converted substrate or µmol product formed/min/mg protein) with regard to the substrate used, broad substrate specificity, and the resulting product has a high degree of enantiomeric purity. In the context of this description, the expression "a high degree of enantiomeric purity" means that the enantiomer in question is present with at least 95% enantiomeric excess (ee) in the mixture with the other enantiomer, preferably with more than 98% ee. The use of D-LDH from *Staphylococcus epidermidis, Leuconostoc mesonteroides,* or *Lactobacillus leichmannii* as the α-ketoacid dehydrogenase and an α-oxo-carboxylic acid as the substrate also results in high rates of production and good yields of the product D- α-hydroxy-carboxylic acid. Consequently, its use reduces the overall expense of this process which is of great importance and a considerable economic advantage if the enzymatic conversions are carried out on a large scale. For these reasons these sources for the enzyme are preferred. In the analogous process in which the substrate is first oxidized with L-amino acid oxidase and the resulting product reduced with the enzyme L-lactate dehydrogenase from bovine heart, the same benefits in the preparation of L-α-hydroxy- carboxylic acids, hydrates, and salts thereof are observed.

The electron donor used for the D-lactate dehydrogenase from *Staphylococcus epidermidis, Leuconostoc mesenteroides,* or *Lactobacillus leichmannii* or for the L-lactate-dehydrogenase from bovine heart is preferably the coenzyme nicotinamide adenine dinucleotide in its reduced form. (NADH) which is oxidized by the D- or L-LDH to NAD. For the regeneration of the coenzyme, an enzyme/substrate system consisting of a NADH-recycling enzyme and its substrate, e.g. a formate dehydrogenase(FDH)/formate system; a alcohol dehydrogenase(ADH)/ethanol, isopropanol, cyclohexanol system; a glucose dehydrogenase (GDH)/glucose system; or the like system, is used. These systems produce CO₂H-CO₃-and acetaldehyde, respectively, as byproducts. A formate dehydrogenase (FDH)/ formate system in which a salt of formic acid, for example an alkali metal formate, e.g. potassium or sodium formate, is used as the formate source is preferred. Suitable formate dehydrogenases for carrying out the process of the invention can be isolated, for example from *Candida boidinii* or from *Pseudomonas oxalacticus.* The dehydrogenases are suitably added in such an amount that the ratio of the activities of formate dehydrogenase and α-keto acid dehydrogenase is between 1:1 and 1:100.

A broad range of amino-carboxylic acids or salts thereof may be readily converted into either a L- or D-oc-hydroxy-carboxylic acids or salt thereof depending on the choice of conditions employed for enzymatic conversion (L- or D-LDH). Preferred compounds of the subject invention are amino-carboxylic acids, hydrates, or salts thereof of having the following structural formula: 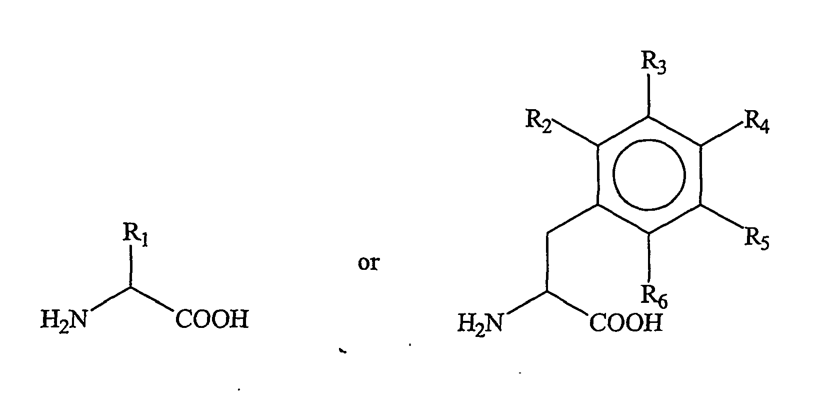 wherein;
R¹ is hydrogen or straight or branched C₁-C₉ alkyl independently substituted with one or more substituent selected from the group consisting of halo, amino, nitro, cyano, carboxy, phosphonyl, sulfonyl, thioacetyl, thiopropionyl, phenyl, C₁-C₆ cycloalkyl, thienyl, hydroxyl, naphthyl, pyridinyl, amino, aminoalkylthio, carboxyalkylthio, carboxyaminoalkylthio, guanidino, nitroguanidino, ureido, aminooxy, guanidinoxy, OH, C₁-C₃ alkoxy, benzyloxy, 3-(2,3-benzopyrrole), 3-(5-hydroxy-2,3-benzopyrrole), 3-(5-fluoro-2,3-benzopyrrole), 3-(benzothiophene), methylimidazyl, amido, 3-anthraniloyl, N-(3-hydroxy-4-pyridone), ribosyladenosinealkylthio, C₁-C₄ alkylthio, benzylthio, *p*-methoxybenzylthio, phenylthio, carbamthioyl, cysteindisulfide, alkylseleno, alkylsulfone, alkylsulfoxide, alkylsulfoximyl, vinyl, allyl, propargyl; and
R², R³,R⁴ R⁵ and R⁶, independently, are selected from the group consisting of hydrogen, halo, amino, nitro, cyano, methyl, hydroxy, C₁-C₃ alkoxy, benzyloxy, phosphonyl, and benzoyl.

Through the use of this invention, for example, L-phenylalanine can be converted into L- or D-phenyl lactic acid, L-2-amino-4-methylvaleric acid can be converted into L- or D-2-hydroxy-4-methylvaleric acid, L-2-amino-3-methylvaleric acid can be converted into L- or D- 2-hydroxy-3-methylvaleric acid, L-2-amino-3-methylbutyric acid can be converted into L- or D- 2-hydroxy-3-methylbutyric acid, L-2-aminovaleric acid can be converted into L-or D- 2-hydroxyvaleric acid, L-2-aminobutanoic acid can be converted into L-or D-2-hydroxybutanoic acid, L-norvaline can be converted into L- or D-2-hydroxypentanoic acid, L-norleucine can be converted into L- or D- 2-hydroxyhexanoic acid, L-valine can be converted into L- or D-3-methyl-2-hydroxybutanoic acid, L-leucine can be converted into L- or D-4-methyl-2-hydroxypentanoic acid, L-t-butylalanine can be converted into L- or D-4,4-dimethyl-2-hydroxypentanoic acid, L-serine can be converted into L- or D-3-hydroxy-2-hydroxypropionic acid, L-homophenylalanine can be converted into L- or D-2-hydroxy-4-phenylbutanoic acid, L-1-naphthylalanine can be converted into L- or D- 3-(1-naphthyl)-2-hydroxypropionic acid, L-2-naphthylalanine can be converted into L- or D- 3-(2-naphthyl)-2-hydroxypropionic acid, L-tyrosine can be converted into L- or D-*p-*hydroxyphenyl-2-hydroxypropionic acid, L-*p*-F-phenylalanine can be converted into L- or D-*p*-F-phenyl- 2-hydroxypropionic acid, L-glutamic acid can be converted into L- or D- α-hydroxyglutaric acid, L-2-aminoadipic acid can be converted into L- or D- 2-hydroxyadipic acid, L-citrulline can be converted into L- or D-2-hydroxy-5-ureidovaleric acid, L-homocitrulline can be converted into L- or D- 2-hydroxy-6-ureidonohexanoic acid, L-cysteine can be converted into L- or D- mercapto-2-hydroxypropionic acid, L-tryptophan can be converted into L- or D- indole-3-(2-hydroxy)propionic acid. Suitably, the reacting α-aminocarboxylic acids, e.g., phenylalanine are employed in the form of their salts, e.g., phenylalanine hydrochloride, or the like.

Numerous types of reaction vessels can be used, such as fermentors, stirred reactors, fixed bed reactors, fluidized bed reactors or membrane reactors (see Hartmeier, "Immobilisierte Biokatalysatoren", Berlin 1986 for a summary). An air sparge is attached to the reaction vessel to feed in O₂ as a substrate. In the batch process, the fermentor is supplied with an aqueous solution of the substrate α-amino-carboxylic acid, for example, preferably in the form of its potassium or sodium salt. The concentration of the substrate should amount to 50 to 100% of the maximal possible concentration, however, it is not permitted to exceed 2,000 mmol/l, preferably between 20 and 1,000 mmol/l, and especially of 100-500 mM, is preferred. This is incubated while stirring under O₂ with the amino acid deaminase from *P. myxofaciens* advantageously used in such a quantity that the ratio of the activities of the deaminase enzyme and α-keto-acid dehydrogenase is from 1:0.1 to 1:100. The fermentor is then supplied with an aqueous solution of coenzyme NAD(H) in a concentration of from 0.01 to 10 mM, preferable of approximately 0.1 mM, the NADH-recycling enzyme, for example an alcohol dehydrogenase or a formate dehydrogenase, and ethanol or formate, respectively, in a concentration of from 100 to 1200 mM, preferable of approximately 300 mM, and with the D-lactate dehydrogenase from *Staphylococcus epidennidis, Leuconostoc mesenteroides,* or *Lactobacillus leichmannii* or with the L-lactate dehydrogenase from bovine heart until conversion is complete. The enzymes are advantageously used in such quantities that the ratio of the activities of NADH-recycling enzyme and substrate-specific dehydrogenase is from 1:0.1 to 1:10. The coenzyme required is used in the form of NAD(H) of which the molecular weight has not been increased, i.e. native NAD(H), in a concentration of from 0.01 to 10 mM, preferably of 0.1 mM, although the use of NAD(H) that has been bonded to a polyethylene glycol in order to increase the molecular weight may also be used. The concentration of formate ions or ethanol is between 100 and 6,000 mmol/l, preferably between 300 and 2,000 mmol/l for formate. The reaction mixture has a pH in the range of from pH 6 to 9, e.g. pH 7.5, as is customary for enzymatic reactions. The reaction temperature is from 20° C to 40° C., preferably around room temperature. The reaction is sparged with N₂ to prevent oxidation of the enzymes and to help drive off CO₂. The product is crystallized from the reaction mixture by the addition of an acid, for example a mineral acid, such as hydrochloric acid, and the like. A preferred process of the invention is one as described above wherein the enzymatic conversion is carried out in an bioreactor such as a fermentor: a) that contains a reaction mixture consisting of a solution of a formate dehydrogenase or an alcohol dehydrogenase, preferably a formate dehydrogenase, the D-lactate dehydrogenase from *Staphylococcus epidermidis, Leuconostoc mesenteroides;* or *Lactobacillus leichmannii* or the L-lactate dehydrogenase from bovine heart, and nicotinamide adenine dinucleotide (NAD(H)) in a concentration of, for example, from 0.01 to 1 mM, preferably of 1 mM; b) to which is fed an aqueous solution of the substrate α-amino- carboxylic acid, for example in the form of its hydrochloride or sodium salt, in a concentration of up to 500 mM, for example in a concentration of from 20 to 500 mM, preferably of

50-200 mM, and formate, for example potassium or sodium formate, or, respectively, ethanol, for example in a concentration of from 100 to 1200 mM, preferably of 300 mM; and c) in which the compound formed is isolated by ion exchange or solvent extraction. The process can comprise, consist essentially of, or consist of the steps set forth and the material employed can comprise, consist essentially of or consist of those stated.

The results of enzyme activity determination with the present substrates are particularly encouraging towards the use of the enzymes on a preparative scale as the unexpectedly high conversion of 70/100% of the substrates which was expected to compromise the success of the chemical conversion in terms of time, yield, enantiomeric purity and cost effectiveness. Product formation can be measured by following the strong absorbance at 340 nm of the reduced cofactor NADH compared to the oxidized cofactor NAD⁺ and the diminution of absorbance with concentration of NADH. The diminution of absorbance, which is directly proportional to the concentration of NADH, may be used to estimate the rate of enzymatic reaction. This technique can be limited by several factors including the purity of the enzyme, where the presence of other enzyme activities may lead to oxidation of NADH activity correlates to the formation of the expected product.

The α-hydroxycarboxylic formed can be obtained/isolated from the filtrate in a known manner. This can be done, for example by separating the α-hydroxy-carboxylic acid from the unreacted α-aminocarboxyuc acid and α-ketocarboxylic acid by means of a basic ion exchanger which make the most of their different acid strengths. The different solubility of salts, particularly calcium salts, in many cases makes possible a separation through fractional crystallization. In a given case the differences in polarity can also be drawn on for separation by extraction with a suitable solvent.

The characterization of α-hydroxy-carboxylic acids, hydrates, and salts thereof and stereochemistry of each reduction can be determined by NMR and capilliary GC analyses of the (+)-MPTA Mosher derivative prepared by esterification of the α-hydroxy-carboxylic acid, followed by acylation with (+)-MTPA-Cl, (Dale, J. A., et al. J. Org. Chem., (1969), 34, 2543) and comparison with a racemic standard. Alternately, the characterization of α-hydroxy-carboxylic acids, hydrates, and salts thereof and stereochemistry of each reduction can be determined by chiral, reverse-phase high performance liquid chromatography (RP-HPLC) analyses. These are the standard literature protocols for chiral analysis of α-hydroxy carboxylic acid derivatives and is sensitive enough to detect < 0.5% of the minor diasteroisomer. Enantiomerically pure, chiral α-hydroxy- carboxylic acids, hydrates, and salts thereof are valuable as inhibitors, pharmaceuticals, therapeutic agents, and as precursors/intermediates in their preparation.

In the following examples the process of the invention will be explained in more detail in connection with the conversion of L-phenylalanine into D-phenyl lactic acid, L-leucine into D-2-hydroxy-4-methylpentanoic acid and L-norvaline into D-2-hydroxypentanoic acid. The following examples are intended to illustrate the present invention without implying any limitation thereof to the scope of the examples..Unless otherwise indicated all parts and percentages are by weight. Although these enzymes can exhibit small variations of amino acid sequences in the periphery of the protein framework, those residues in and around the active site, responsible for catalysis of the α-oxo carboxylic acid/α-hydroxy-carboxylic acid inter-conversion, are usually conserved. For a given α-oxo carboxylic acid, a given enzyme activity may be detected by UV spectroscopy and characterized in terms of Kₘ, and the catalytic turnover, k_{cat}.

### EXAMPLE 1

### L-Amino Acid Oxidase Gene Isolation and Manipulation

The amino acid deaminase gene from *P. myxofaciens* was isolated by whole cell PCR using primers (see Table 2) designed to the published *P*. *mirabilis* DNA sequence. Ligations were carried out using a Takara Biochemicals DNA ligation kit from Panvera (Madison, WI). PCR was carried out using standard conditions in a Perkin Elmer 9600 Thermal Cycler with *Taq* or Ultma DNA polymerase from Perkin Elmer (Norwalk, CT). Oligonucleotides were prepared using an Applied Biosystems 300 B DNA synthesizer.

**Table 2. Primers used to construct plasmid pPT381 (aad 6xHis)**

| Primer | Sequence |
|---|---|
| 5*' aad* MB2171 | 5'-TTT GGA TCC AAA ATG AAC ATC TCT CGT CGT AAA CTG CTG TTA GGT GTT GGT GCT GCG GGC GT-3' *BamHI* |
| *3' aad* 6xHis MB2280 | 5'-AGC TTT GTC GAC GGG CCC TTA_GTG ATG GTG **ATG GTG ATG** CTT CTT AAA ACG ATC CAA AC-3' *Sal*I |

Underline designates the aad gene and bold designates the 6xHis insert at the C-terminus.

### EXAMPLE 2

### Screening Studies for L-Amino Acid Oxidase

Oxygen consumption was measured using a Clark-type O₂ electrode in an Oxygraph system from Hansatech Instruments (Norfolk, England), which was zeroed with dithionite. Amino acid substrates (10.0 mM) were incubated in 50 mM potassium phospate buffer, pH 7.5, at 30°C for 2 minutes in a total volume of 990 µl prior to adding enzyme. Reactions were initiated by adding 10 µl enzyme (104 µg protein) and the O₂ consumption measured for an additional 3 minutes. L-phenylalanine was used as the reference substrate and the linear rate was determined and set to 100%. All other amino acids were compared to L-phenylalanine after a buffer-only blank rate was subtracted.

### EXAMPLE 3

### Screening Studies for Lactate Dehydrogenase

Lactate dehydrogenases were screened on a number of keto acid substrates at 25°C with a SpectraMax 250 microplate reader from Molecular Devices (Sunnyvale, CA). Reactions were carried out by adding 10 µl dilute enzyme to 190 µl of 30 mM keto acid + 0.6 mM NADH in 100 mM potassium phosphate buffer, pH 7.0, and monitoring NADH oxidation at 340 nm. Pyruvate was used as the reference substrate and the enzymes' activities were determined and set to 100%. All other keto acids were compared to pyruvate after adjusting for background activity on a buffer-only blank.

### EXAMPLE 4

### Production of D-Phenyllactic Acid (D-PLA) in the presence of L-amino acid deaminase (L-AAD).

The deaminase reaction was carried out via whole cell biotransformation by adding 100 g (wet cell pellet) of E. *coli,* that was carrying a multi-copy clone of the gene for L-amino acid deaminase, to 500 ml deionized water containing L-phenylalanine (100 mmol). The reaction was mixed rapidly in a 2 liter closed fermentor at 32°C, pH 7.5-8, with oxygen sparged into the system at a rate of 1 v.v.m. Deamination was allowed to continue until dissolved oxygen started to rise (~ 1 hour) at which point oxygen flow was shut off. The dehydrogenation reaction was initiated by adding 500 ml of solution containing sodium formate (100 mmol), mercaptoethanol (1 mmol), dithiothreitol (1 mmol), Tris-CI (25 mmol), nicotinamide adenine dinucleotide (1 mmol), D-lactate dehydrogenase from *S*. *epidermidis* (3,800 units) and formate dehydrogenase from *C*. *boidinii* (380 units). The reaction was carried out at room temperature with nitrogen sparged at 0.05 v.v.m., and pH maintained at 7.5 with 1 N HCI. After 32 hours the titer of D-PLA was 9.24 g/L, which amounted to a 58.1% yield from L-phenylalanine.

### EXAMPLE 5

### Production of D-Phenyllactic Acid (D-PLA) after removal of L-AAD cells.

The deaminase reaction was carried out *via* a whole cell biotransformation by adding 100 g (wet cell pellet) of *E*. *coli,* that was carrying a multi-copy clone of the gene for L-amino acid deaminase, to 500 ml deionized water containing L-phenylalanine (100 mmol). The reaction was mixed rapidly in a 2 liter closed fermentor at 32°C, pH 7.5-8, with oxygen sparged into the system at a rate of 1 v.v.m. Deamination was allowed to continue until dissolved oxygen started to rise (~ 1 hour) at which point oxygen flow was shut off. The L-AAD cells were removed via centrifugation at 8,000 x G for 20 minutes, the supernatant poured into a 2 liter fermentor and allowed to adjust to 22°C. The dehydrogenation reaction was initiated by adding 500 ml of solution containing sodium formate (100 mmol), mercaptoethanol (1 mmol), dithiothreitol (1 mmol), Tris-Cl (25 mmol), nicotinamide adenine dinucleotide (1 mmol), D-lactate dehydrogenase from *S*. *epidermidis* (3,800 units) and formate dehydrogenase from *C*. *boidinii* (380 units). The reaction was carried out at room temperature with nitrogen sparged at 0.05 v.v.m., and pH maintained at 7.5 with 1 N HCL After 31.75 hours the titer of D-PLA was 13.59 g/L, which amounted to a 88.9% yield from L-phenylalanine.

### EXAMPLE 6

### Production of D-2-Hydroxy-4- Methylpentanoic Acid

The deaminase reaction was carried out via a whole cell biotransformation by adding 100 g (wet cell pellet) of *E*. *coli,* that was cloned with the gene for L-amino acid deaminase, to 500 ml deionized water containing L-leucine (100 mmol). The reaction was mixed rapidly in a 2 liter fermentor at 32°C, pH 7.5 - 8, with oxygen sparged into the system at a rate of 1 v.v.m. Deamination was allowed to continue until dissolved oxygen started to rise (~1.5 hours) at which point oxygen flow was shut off. The L-AAD cells were removed via centrifugation at 8,000 x G for 20 minutes, the supernatant poured into a 2 liter fermentor and allowed to adjust to -22°C. The dehydrogenation reaction was initiated by adding 500 ml of solution containing sodium formate (100 mmol), mercaptoethanol (1 mmol), dithiothreitol (1 mmol), Tris-C1 (25 mmol), nicotinamide adenine dinucleotide (1 mmol), D-lactate dehydrogenase from *S. epidermidis* (3,800 units) and formate dehydrogenase from *C*. *boidinii* (380 units). The reaction was carried out at room temperature with nitrogen sparged at 0.05 v.v.m., and pH maintained at 7.5 with 1 N HCI. After 44 hours, 100 ml of acid was added and the titer of D-2-hydroxy 4-methylpentanoic acid was 7.2 g/L, which amounted to a 60.0% yield from L-leucine.

### EXAMPLE 7

### Production of L-2-Hydroxy-4-Methylpentanoic Acid

The deaminase reaction was carried out via a whole cell biotransformation by adding 100 g (wet cell pellet) of E. *coli,* that was cloned with the gene for L-amino deaminase, to 500 ml deionized water containing L-leucine (100 mmol). The reaction was mixed rapidly in a 2 liter fermentor at 32°C, pH 7.5 - 8, with oxygen sparged into the system at a rate of 1 v.v.m. Deamination was allowed to continue until dissolved oxygen started to rise. (~1.5 hours) at which point oxygen flow was shut off. The L-AAD cells were removed via centrifugation at 8,000 x G for 20 minutes, the supernatant poured into a 2 liter fermentor and allowed to adjust to ~22°C. The dehydrogenation reaction was initiated by adding 500 ml of solution containing sodium formate (100 mmol), mercaptoethanol (1 mmol), dithiothreitol (1 mmol), Tris-C1 (25 mmol), nicotinamide adenine dinucleotide (1 mmol), L-lactate dehydrogenase from rabbit muscle (3,800 units) and formate dehydrogenase from *C*. *boidinii* (380 units). The reaction was carried out at room temperature with nitrogen sparged at 0.05 v.v.m., and pH maintained at 7.5 with 1 N HCI. After 44 hours, 8 ml of acid was added and the titer of L-2-hydroxy-4-methylpentanoic acid was 1.4 g/L, which amounted to a 10.6% yield from L-leucine.

### EXAMPLE 8

### Production of D-2-Hydroxypentanoic Acid

The deaminase reaction was carried out via a whole cell biotransformation by adding 100 g (wet cell pellet) of *E*. *coli,* that was cloned with the gene for L-amino acid deaminase to 500ml deionized water containing L-norvaline (100 mmol). The reaction was mixed rapidly in a 2 liter fermentor at 32°C, pH 7.5 - 8, with oxygen sparged into the system at a rate of 1 v.v.m. Deamination was allowed to continue until the dissolved oxygen started to rise (3 hours) at which point oxygen flow was shut off. The L-AAD cells were removed via centrifugation at 8,000 x G for 20 minutes, the supernatant poured into a 2 liter fermentor and allowed to adjust to ~22°C. The dehydrogenation reaction was initiated by adding 500 ml of solution containing sodium formate (100 mmol), mercaptoethanol (1 mmol), dithiothreitol (1 mmol), Tris-C1 (25 mmol), nicotinamide adenine dinucleotide (1 mmol), D-lactate dehydrogenase from *S*. *epidermidis* (3,800 units) and formate dehydrogenase from *C*. *boidinii* (380 units). The reaction was carried out at room temperature with nitrogen sparged at 0.05 v.v.m., and pH maintained at 7.5 with 1 N HCI. After 44 hours, 89 ml of acid was added and the titer of D-2-hydroxypentanoic acid was 7.96 g/L, which amounted to a 73.4% yield from L-norvaline.
## Claims

1. A method for the production by a batch process of a chiral α-hydroxycarboxylic acid, hydrate, or salt thereof comprising oxidizing an α-amino-carboxylic acid, hydrate, or salt thereof (the substrate) using a L-amino acid deaminase or oxidase; and subsequently reducing the product thereof using a dehydrogenase; wherein the concentration of the substrate is between 0.1 and 2 mol/litre.

2. The method in accordance with claim 1, wherein said dehydrogenase is a L-lactate dehydrogenase (EC#1.1.1.28) or a D-lactate dehydrogenase (EC#1.1.1.27).

3. The method in accordance with claim 1 wherein said dehydrogenase is a L- or D-lactate dehydrogenase the source of which is selected from the group consisting of bovine heart, rabbit muscle, porcine heart, porcine muscle, *Staphylococcus epidermidis, Leuconostoc mesenteroides, Lactobacillus leichmannii, or Bacillus stearothermophilus.*

4. The method in accordance with claim 1, wherein the source of said L-amino acid deminase or oxidase is selected from the group consisting of *Proteus myxofaciens, Proteus rettgeri, Cellulomonas cellulans* AM8, *Bacillus carotarum* 2Pfa, *Proteus vulgaris, Proteus mirabilis, Morganella morganii, Pseudomonas sp.* P-501, *Providencia sp.* PCM 1298, *Providencia alcalifaciens, Corynebacterium, Calloselasma rhodostoma, Crotalus terrificus terrifi cus, Crotalus adamanteus, Crotalus atrox, Agkistrodon piscivorus piscivorous, Trimeresurus mucrosquamantus, Ophiophagus hannah, Naja naja kaouthia, Synechococcus* PCC 7942, *Synechococcus* PCC 6301, *Anacystis nidulans, Amphiora crassissima, Gymnogongrus flabe3Z.zform.is, Chlamydomonas reinhardtii, Pleurochrysis, Neisseria meningitidis, Trachoderma viride* Y244-2, and *Neurospora crassa.*

5. The method in accordance with claim 1, wherein the source of said L-amino acid deaminase or oxidase is *E. coli,* that was cloned with the gene for L-amino deaminase.

6. The method in accordance with claim 1, wherein said chiral α-hydroxycarboxylic acid salt is a sodium or potassium salt.

7. The method in accordance with claim 1, wherein the chiral α-hydroxy-carboxylic acid is a substituted or unsubstituted (2R)- or(2S)- 2-hydroxyalkanoic acid.

8. The method in accordance with claim 1, wherein the chiral α-hydroxy-carboxylic acid is selected from the group consisting of (2R)- or (2S)- 2-hydroxybutanoic acid, (2R)- or (2S)- 2-hydroxypentanoic acid, (2R)- or (2S)-2-hydroxyhexanoic acid, (2R) - or (2S) - 2-hydroxy-3-methylpentanoic acid, (2R)- or (2S)- 2-hydroxy-4-pentanoic acid, (2R)- or (2S)- 2,3-dihydroxypropanoic acid, (2R)-or (2S)- 2,4- dihydroxypropanoic acid, (2R)- or (2S)-3-chloro-2-hydroxypropanoic acid, (2R)- or (2S)- 2-hydroxy-3-mercaptopropanoic acid, (2R)- or (2S)- 2-hydroxy-4-thiomethylbutanoic acid, (2R)- or (2S)- phenyllactic acid, (2R) - or (2S) - p-hydroxyphenyllactic acid, (2R) - or (2S) - 2-hydroxy-4-phenylbutanoic acid, (2R)- or (2S)- 2-hydroxy-3-indolylpropanoic acid, (2R) - or (2S)- 3-methyl-2-hydroxybutanoic acid, (2R)- or (2S)- 4-methyl-2-hydroxypentanoic acid, (2R)- or (2S)- 4,4-dimethyl-2-hydroxypentanoic acid, (2R)- or (2S)- 3-hydroxy-2-hydroxypropionic acid, (2R)- or (2S)- 2-hydroxy-4-phenylbutanoic acid, (2R)- or (2S)- 3-(2-pyridyl)-2-hydroxypropionic acid, (2R)- or (2S)- 3-(3-pyridyl)-2-hydroxypropionic acid, (2R)- or 2S)- 3-(4-pyridyl)-2-hydroxypropionic acid, (2R)- or (2S)- 3-(1-naphthyl)-2-hydroxypropionic acid, (2R)- or (2S)- 3-(2-naphthyl)-2-hydroxypropionic acid, (2R)- or(2S)- p-hydroxyphenyl-2-hydroxypropionic acid, (2R)- or (2S)- *p*-F-phenyl-2-hydroxypropionic acid, (2R)- or (2S)- α-hydroxyglutaric acid, (2R)- or (2S)- 2-hydroxyadipic acid, (2R)- or (2S)-2-hydroxy-5-ureidovaleric acid, (2R)- or (2S)- 2-hydroxy-6-ureidonohexanoic acid, (2R)- or (2S)- mercapto-2-hydroxypropionic acid, (2R)- or (2S)- indole-2-hydroxypropionic acid, and a combination thereof.

9. The method in accordance with claim 1, wherein the α-amino-carboxylic acid, hydrate, or salt thereof has the following structural formula: 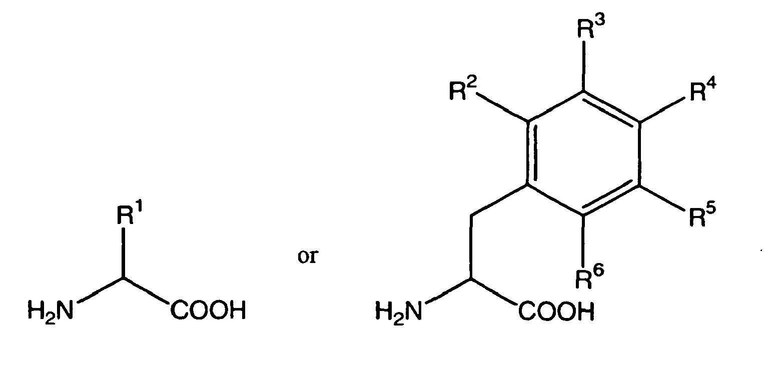 wherein;
R¹ is hydrogen or straight or branched C₁-C₉ alkyl independently substituted with one or more substituent selected from the group consisting of halo, amino, nitro, cyano, carboxy, phosphonyl, sulfonyl, thioacetyl, thiopropionyl, phenyl, C₁-C₆ cycloalkyl, thienyl, hydroxyl, naphthyl, pyridinyl, amino, aminoalkylthio, carboxyalkylthio, carboxyanminoalkylthio, guanidino, nitroguanidino, ureido, aminooxy, guanidinoxy; OH, C₁-C₃ alkoxy, benzyloxy, 3-(2,3-benzopyrrole), 3-(5-hydroxy-2,3-benzopyrrole), 3-(5-fluoro-2,3-benzopyrrole), 3-(benzothiophene), methylimidazyl, amido, 3-anthraniloyl, N-(3-hydroxy-4-pyridone), ribosyladenosinealkylthio, C₁-C₄ alklythio, benzylthio, *p*-methoxybenzylthio, phenylthio, carbamthioyl, cysteindisulfide, alkylseleno, alkylsulfone, alkylsulfoxide, alkylsulfoximyl, vinyl, allyl, propargyl; and
R²,R³,R⁴,R⁵ and R⁶, independently, are selected from the group consisting of hydrogen, halo, amino, nitro, cyano, methyl, hydroxyl, C₁-C₃ alkoxy, benzyloxy, phosphonyl, and benzoyl.

10. The method in accordance with claim 1 which comprises preparing said chiral α-hydroxy-carboxylic acid in an enantiomeric purity of more than 95% enantiomeric excess.

11. The method in accordance with claim 1, wherein said reducing step is effected in combination with an electron donor and an enzyme/substrate system for recyling the electron donor.

12. The method in accordance with claim 11, wherein said electron donor is nicotinamide adenine dinucleotide and said enzyme/substrate system for recycling the electron donor is formate dehydrogenase/formate.

13. The method in accordance with claim 1, wherein said oxidizing step is carried out with whole cells.

14. The method in accordance with claim 1, which is carried out in a reaction vessel or fermentor:
a) that is equipped with any oxygen sparge;
b) that contains a reaction mixture consisting of a solution of *E. coli,* that was cloned with the gene for L-amino acid deaminase (oxidizing enzyme) and an α-amino-carboxylic acid;
c) to which are fed an aqueous solution of a formate ion (cobustrate), mercaptoethanol, dithiothreitol (antioxidants), Tris-CI (buffer), nicotinamide adenine dinucleotide (electron donor), D-lactate dehydrogenase (reducing enzyme) and fomrate dehydrogenase (enzyme/substrate system for regenerating/recycling the electron donor); and
d) in which the compound formed is isolated by ion exchange or solvent extraction.

15. A use of α-amino-carboxylic acids, hydrates, and salts thereof as substrates for a coupled enzyme system comprising a L-amino acid deaminase or oxidase and a dehydrogenase, for the production by a batch process of α-hydroxy-carboxylic acids, hydrates, and salt thereof, wherein the substrate concentration is between 0.1 and 2 mol/litre.

## Patentansprüche

1. Ein Verfahren zur Herstellung einer chiralen α-Hydroxycarbonsäure, eines Hydrats oder Salzes davon mittels eines Batch-Prozesses, welches das Oxidieren einer α-Aminocarbonsäure, eines Hydrats oder Salzes davon (das Substrat) unter Verwendung einer L-Aminosäuredeaminase oder -oxidase; und das anschließende Reduzieren dieses Produkts unter Verwendung einer Dehydrogenase umfasst; wobei die Konzentration des Substrats zwischen 0,1 und 2 Mol/l liegt.

2. Das Verfahren gemäß Anspruch 1, wobei die Dehydrogenase eine L-Laktat-Dehydrogenase (EC#1.1.1.28) oder eine D-Laktat-Dehydrogenase (EC#1.1.1.27) ist.

3. Das Verfahren gemäß Anspruch 1, wobei die Dehydrogenase eine L- oder D-Laktatdehydrogenase aus einer Quelle ist, welche aus der aus dem Folgenden bestehenden Gruppe ausgewählt ist: Rinderherz, Kaninchenmuskel, Schweinherz, Schweinemuskel, *Staphylococcus epidermidis, Leuconostoc mesenteroides, Lactobacillus leichmannii oder Bacillus stearothermophilus.*

4. Das Verfahren gemäß Anspruch 1, wobei die Quelle der L-Aminosäuredeaminse oder -oxidase ausgewählt ist aus der aus dem Folgenden bestehenden Gruppe: *Proteus myxofaciens, Proteus rettgeri, Cellulomonas cellulans AM8, Bacillus carotarum 2Pfa, Proteus vulgaris, Proteus mirabilis, Morganella morganii, Pseudomonas sp. P-501, Providencia sp. PCM 1298, Providencia alcalifaciens, Corynebacterium, Calloselasma rhodostoma, Crotalus terri ficus terrificus, Crotalus adamanteus, Crotalus atrox, Agkistrodon piscivorus piscivorous, Trimeresurus mucrosquamantus, Ophiophagus hannah, Naja naja kaouthia, Synechococcus PCC 7942, Synechococcus PCC 6301, Anacystis nidulans, Amphiora crassissima, Gymnogongrus flabelliformis, Chlamydomonas reinhardtii, Pleurochrysis, Neisseria meningi tidis, Trichoderma viride Y244-2* und *Neurospora crassa.*

5. Das Verfahren gemäß Anspruch 1, wobei die Quelle der L-Aminosäuredeaminase oder -oxidase *E.coli* ist, welches mit dem Gen für L-Aminodeaminase geklont worden ist.

6. Das Verfahren gemäß Anspruch 1, wobei das chirale α-Hydroxycarbonsäuresalz ein Natrium- oder Kaliumsalz ist.

7. Das Verfahren gemäß Anspruch 1, wobei die chirale α-Hydroxycarbonsäure eine substituierte oder unsubstituierte (2R)- oder (2S)-2-Hydroxyalkanoesäure ist.

8. Das Verfahren gemäß Anspruch 1, wobei die chirale α-Hydroxycarbonsäure aus der aus dem Folgenden bestehenden Gruppe ausgewählt ist: (2R)- oder (2S)-2-Hydroxybuttersäure, (2R)- oder (2S)-2-Hydroxypentansäure, (2R)- oder (2S)-2-Hydroxyhexansäure, (2R)- oder (2S)-2-Hydroxy-3-methylpentansäure, (2R)- oder (2S)-2-Hydroxy-4-pentansäure, (2R)- oder (2S)-2,3-Dihydroxypropansäure, (2R)- oder (2S)-2,4-Dihydroxypropansäure, (2R)- oder (2S)-3-Chlor-2-hydroxypropansäure, (2R)- oder (2S)-2-Hydroxy-3-mercaptopropansäure, (2R)- oder (2S)-2-Hydroxy-4-thiomethylbuttersäure, (2R)- oder (2S)-Phenylmilchsäure, (2R)- oder (2S)-p-Hydroxyphenylmilchsäure, (2R)- oder (2S)-2-Hydroxy-4-phenylbuttersäure, (2R)- oder (2S)-2-Hydroxy-3-indolylpropansäure, (2R)- oder (2S)-3-Methyl-2-hydroxybuttersäure, (2R)- oder (2S)-4-Methyl-2-hydroxypentanosäure, (2R)- oder (2S)-4,4-Dimethyl-2-hydroxypentansäure, (2R)- oder (2S)-3-Hydroxy-2-hydroxypropionsäure, (2R)- oder (2S)-2-Hydroxy-4-phenylbuttersäure, (2R)- oder (2S)-3-(2-Pyridyl)-2-hydroxypropionsäure, (2R)- oder (2S)-3-(3-Pyridyl)-2-hydroxypropionsäure, (2R)- oder (2S)-3-(4-Pyridyl)-2-hydroxypropionsäure, (2R)- oder (2S)-3-(1-Naphthyl)-2-hydroxypropionsäure, (2R)- oder (2S)-3-(2-Naphthyl)-2-hydroxypropionsäure, (2R)- oder (2S)-p-Hydroxyphenyl-2-hydroxypropionsäure, (2R)- oder (2S)-p-F-Phenyl-2-hydroxypropionsäure, (2R)- oder (2S)-α-Hydroxyglutarsäure, (2R)- oder (2S)-2-Hydroxyadipinsäure, (2R)-oder (2S)-2-Hydroxy-5-ureidovalerainsäure, (2R)- oder (2S)-2-Hydroxy-6-ureidonohexansäure, (2R)- oder (2S)-Mercapto-2-hydroxypropionsäure, (2R)- oder (2S)-Indol-2-hydroxypropionsäure und eine Kombination davon.

9. Das Verfahren gemäß Anspruch 1, wobei die α-Aminocarbonsäure, das Hydrat oder das Salz davon die folgende Strukturformel aufweist: 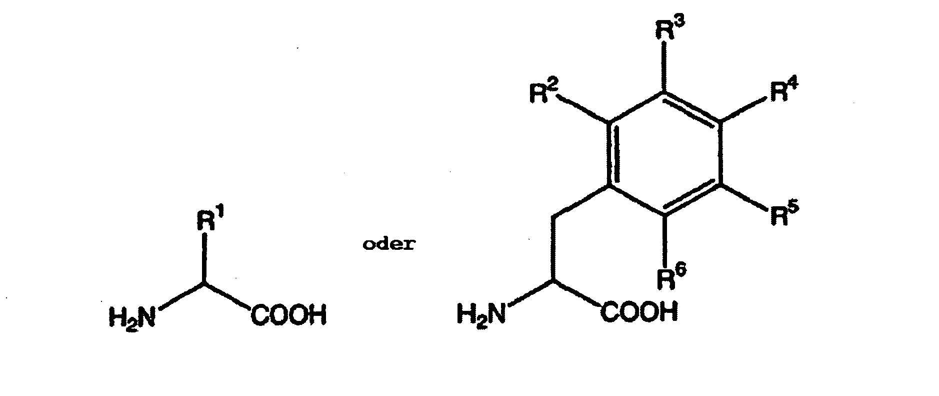 worin;
R¹ ein Wasserstoff oder lineares oder verzweigtes C₁-C₉-Alkyl ist, welches unabhängigerweise mit einem oder mehreren Substituenten substituiert ist, welche aus der aus dem Folgenden bestehenden Gruppe ausgewählt sind: Halo, Amino, Nitro, Cyano, Carboxy, Phosphonyl, Sulfony, Thioacetyl, Thiopropionyl, Phenyl, C₁-C₆-Cycloalkyl, Thienyl, Hydroxyl, Naphthyl, Pyridinyl, Amino, Aminoalkylthio, Carboxyalkylthio, Carboxyaminoalkylthio, Guanidino, Nitroguanidino, Ureido, Aminooxy, Guanidinoxy, OH, C₁-C₃-Alkoxy, Benzyloxy, 3(2,3-Benzopyrrol), 3(5-Hydroxy-2,3-benzopyrrol), 3-(5-Fluor-2,3-benzopyrrol), 3-(Benzothiophen), Methylimidazyl, Amido, 3-Anthraniloyl, N-(3-Hydroxy-4-pydridon), Ribosyladenosinalkylthio, C₁-C₄-Alkylthio, Benzylthio, p-Methoxybenzylthio, Phenylthio, Carbamthioyl, Cysteindisulfid, Alkylseleno, Alkylsulfon, Alkylsulfoxid, Alkylsulfoximyl, Vinyl, Allyl, Propargyl und
R², R³, R⁴, R⁵ und R⁶ unabhängigerweise aus der aus Wasserstoff, Halo, Amino, Nitro, Cyano, Methyl, Hydroxyl, C₁-C₃-Alkoxy, Benzyloxy, Phosphonyl und Benzoyl bestehenden Gruppe ausgewählt sind.

10. Das Verfahren gemäß Anspruch 1, welches das Herstellen der chiralen α-Hydroxycarbonsäure in einer Enantiomerenreinheit von mehr als 95% Enantiomerenüberschuss umfasst.

11. Das Verfahren gemäß Anspruch 1, wobei der Reduzierschritt in Kombination mit einem Elektronendonor und einem Enzym/Substrat-System für die Rückgewinnung des Elektronendonors bewirkt wird.

12. Das Verfahren gemäß Anspruch 11, wobei der Elektronendonor Nikotinamidadenindinukleotid ist und das Enzym/Substrat-System für das Rückgewinnen des Elektronendonors Formiatdehydrogenase/Formiat ist.

13. Das Verfahren gemäß Anspruch 1, wobei der Oxidierschritt mit ganzen Zellen durchgeführt wird.

14. Das Verfahren gemäß Anspruch 1, welches in einem Reaktionskessel oder Fermentor durchgeführt wird:
a) der mit einem Sauerstoffzerstäuber ausgestattet ist;
b) der eine Reaktionsmischung enthält, bestehend aus einer Lösung aus *E.coli*, das mit dem Gen für L-Aminosäuredeaminase (Oxidationsenzym) geklont worden ist, und einer α-Aminocarbonsäure;
c) zu welchen eine wässrige Lösung aus einem Formiation (Cosubstrate), Mercaptoethanol, Dithiothreitol (Antioxidans), Tris-CI (Puffer), Nikotinamidadenindinukleotid (Elektronendonor), D-Laktatdehydrogenase (Reduktionsenzym) und Formiatdehydrogenase (Enzym/Substrat-System für die Regenerierung/Rückgewinnung des Eleketronendonors) zugeführt wird; und
d) in welchem die erzeugte verbindung mittels Ionenaustausch oder Lösungsmittelextraktion isoliert wird.

15. Verwendung von α-Aminocarbonsäuren, Hydraten und Salzen davon als Substrate für ein gekoppeltes Enzymsystem, umfassend eine L-Aminosäuredeaminse oder -oxidase und eine Dehydrogenase, für die Herstellung von α-Hydroxycarbonsäuren, Hydraten und Salzen davon mittels eines Batch-Prozesses, wobei die Substratkonzentration zwischen 0,1 und 2 Mol/l liegt.

## Revendications

1. Procédé pour la production par un traitement par lots d'un acide α-hydroxycarboxylique chiral, d'un hydrate ou d'un sel de celui-ci, comprenant l'oxydation d'un acide α-aminocarboxylique, d'un hydrate ou d'un sel de celui-ci (le substrat) en utilisant une L-aminoacide désaminase ou oxydase et par la suite la réduction du produit de celle-ci en utilisant une déshydrogénase, dans lequel la concentration en substrat se situe entre 0,1 et 2 mol/litre.

2. Procédé selon la revendication 1, dans lequel ladite déshydrogénase est une L-lactate déshydrogénase (EC#1.1.1.28) ou une D-lactate déshydrogénase (EC#1.1.1.27).

3. Procédé selon la revendication 1, dans lequel ladite déshydrogénase est une L-ou D-lactate déshydrogénase, dont la source est choisie dans le groupe comprenant un coeur bovin, un muscle de lapin, un coeur porcin, un muscle porcin, *Staphylococcus epidermidis, Leuconostoc mesenteroides, Lactobacillus leichmannii* ou *Bacillus stearothermophilus.*

4. Procédé selon la revendication 1, dans lequel la source desdites L-aminoacide désaminase ou oxydase est choisie dans le groupe consistant en *Proteus myxofaciens, Proteus rettgeri, Cellulomonas cellulans* AM8, *Bacillus carotarum* 2Pfa, *Proteus vulgaris, Proteus mirabilis, Morganella morganii, Pseudomonas sp.* P-501, *Providencia sp.* PCM 1298, *Providencia alcalifaciens, Corynebacterium, Calloselasma rhodostoma, Crotalus terrificus terrificus, Crotalus adamenteus, Crotalus atrox, Agkistrodon piscivorus piscivorus, Trimeresurus mucros quamantus, Ophiophagus hannah, Naja naja kaouthia, Synechococcus* PCC 7942, *Synechococcus* PCC 6301, *Anacystis nidulans, Amphiora crassissima, Gymnogongrus flabelliformis, Chlamydomonas reinhardtii, Pleurochrysis, Neisseria meningitidis, Trichoderma viride* Y244-2 et *Neurospora crassa.*

5. Procédé selon la revendication 1, dans lequel la source desdites L-aminoacide désaminase ou oxydase est *E. coli,* qui est clonée avec le gène codant pour la L-aminoacide désaminase.

6. Procédé selon la revendication 1, dans lequel ledit sel d'acide α-hydroxycarboxylique chiral est un sel de sodium ou de potassium.

7. Procédé selon la revendication 1, dans lequel l'acide α-hydroxycarboxylique chiral est un acide (2R)- ou (2S)-2-hydroxyalcanoïque substitué ou non substitué.

8. Procédé selon la revendication 1, dans lequel l'acide α-hydroxycarboxylique chiral est choisi dans le groupe consistant en acide (2R)- ou (2S)-2-hydroxybutanoïque, acide (2R)- ou (2S)- 2-hydroxypentanoïque, acide (2R)- ou (2S)-2-hydroxyhexanoïque, acide (2R)- ou (2S)-2-hydroxy-3-méthylpentanoïque, acide (2R)- ou (2S)-2-hydroxy-4-pentanoïque, acide (2R) ou (2S)-2,3-dihydroxypropanoïque, acide (2R) ou (2S)-2,4-dihydroxypropanoïque, acide (2R)-ou (2S)-3-chloro-2-hydroxypropanoïque, acide (2R)- ou (2S)-2-hydroxy-3-mercaptopropanoïque, acide (2R)- ou (2S)-2-hydroxy-4-thiométhylbutanoïque, acide (2R)- ou (2S)-phényllactique, acide (2R)- ou (2S)-p-hydroxyphényllactique, acide (2R)- ou (2S)-2-hydroxy-4-phénylbutanoïque, acide (2R)- ou (2S)-2-hydroxy-3-indolylpropanoïque, acide (2R) -ou (2S)-3-méthyl-2-hydroxybutanoïque, acide (2R)-ou (2S)-4-méthyl-2-hydroxypentanoïque, acide (2R)- ou (2S)-4,4-diméthyl-2-hydroxypentanoïque, acide (2R)- ou (2S)-3-hydroxy-2-hydroxypropionique, acide (2R)- ou (2S)-2-hydroxy-4-phénylbutanoïque, acide (2R)- ou (2S)-3-(2-pyridyl)-2-hydroxypropionique, acide (2R)- ou (2S)-3-(3-pyridyl)-2-hydroxypropionique, acide (2R)- ou (2S)-3-(4-pyridyl)-2-hydroxypropionique, acide (2R)- ou (2S)-3-(1-naphthyl)-2-hydroxypropionique, acide (2R)- ou (2S)-3-(2-naphthyl)-2-hydroxypropionique, acide (2R)- ou (2S)-p-hydroxyphényl-2-hydroxypropionique, acide (2R)- ou (2S)-p-F -phényl-2-hydroxypropionique, acide (2R)- ou (2S)-α-hydroxyglutarique, acide (2R)- ou (2S)-2-hydroxyadipique, acide (2R)- ou (2S)-2-hydroxy-5-uréidovalérique, acide (2R)- ou (2S)-2-hydroxy-6-uréidohexanoïque, acide (2R)- ou (2S)-mercapto-2-hydroxypropionique, acide (2R)- ou (2S)-indole-2-hydroxypropionique et une combinaison de ceux-ci.

9. Procédé selon la revendication 1, dans lequel l'acide α-aminocarboxylique, l'hydrate ou le sel de celui-ci, possède la formule développée suivante : 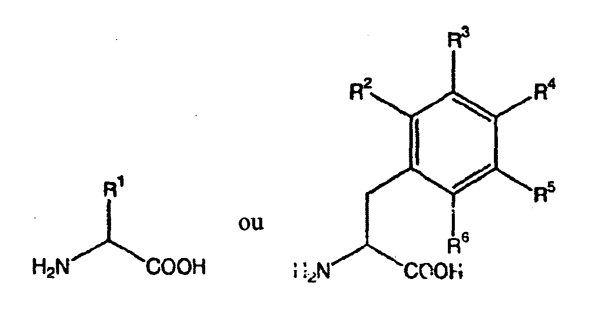 dans laquelle ;
R¹ est un hydrogène ou un alkyle en C₁-C₉ à chaîne droite ou ramifiée, indépendamment substitué par un ou plusieurs substituants choisis dans le groupe consistant en halogéno, amino, nitro, cyano, carboxy, phosphonyle, sulfonyle, thioacétyle, thiopropionyle, phényle, cycloalkyle en C₁-C₆, thiényle, hydroxyle, naphthyle, pyridinyle, amino, aminoalkylthio, carboxyalkylthio, carboxyaminoalkylthio, guanidino, nitroguanidino, uréido, aminooxy, guanidinooxy, OH, alcoxy en C₁-C₃, benzyloxy, 3-(2,3-benzopyrrole), 3-(5-hydroxy-2,3-benzopyrrole), 3-(5-fluoro-2,3-benzopyrrole), 3-(benzothiophène), méthylimidazyle, amido, 3-anthraniloyle, N-(3-hydroxy-4-pyridone), ribosyladénosinealkylthio, alkylthio en C₁-C₄, benzylthio, *p*-méthoxybenzylthio, phénylthio, carbamthioyl, cystéinedisulfure, alkylséléno, alkylsulfone, alkylsulfoxyde, alkylsulfoximyle // sulfoximido, vinyle, allyle, propargyle ; et
R², R³, R⁴, R⁵et R⁶, indépendamment, sont choisis dans le groupe consistant en hydrogène, halogéno, amino, nitro, cyano, méthyl, hydroxyle, alcoxy en C₁-C₃, benzyloxy, phosphonyle et benzoyle.

10. Procédé selon la revendication 1 qui comprend la préparation dudit acide α-hydroxycarboxylique chiral avec une pureté énantiomérique de plus de 95% d'excès énantiomérique.

11. Procédé selon la revendication 1, dans lequel ladite étape de réduction est effectuée en combinaison avec un électrodonneur et un système enzyme/substrat pour recycler l'électrodonneur.

12. Procédé selon la revendication 11, dans lequel ledit électrodonneur est le nicotinamide adénine dinucléotide et ledit système enzyme/substrat pour recycler l'électrodonneur est le formiate déshydrogénase/formiate.

13. Procédé selon la revendication 1, dans lequel ladite étape d'oxydation est effectuée avec des cellules entières.

14. Procédé selon la revendication 1 qui est effectué dans un réacteur ou un fermenteur :
a) est équipé avec n'importe quel injecteur d'oxygène ;
b) contient un mélange réactionnel consistant en une solution d'E. coli, qui a été clonée avec le gène codant pour la L-aminoacide désaminase (enzyme d'oxydation) et un acide α-aminocarboxylique ;
c) qui est alimenté avec une solution aqueuse d'un ion formiate (cosubstrat), de mercaptoéthanol, de dithiothréitol (antioxydants), de Tris-Cl (tampon), de nicotinamide adénine dinucléotide (électrodonneur), de D-lactate déshydrogénase (enzyme de réduction) et de formiate déshydrogénase (système enzyme/substrat pour la régénération/recyclage de l'électrodonneur) et
d) dans lequel le composé formé est isolé par échange ionique ou par extraction par solvant.

15. Utilisation d'acides α-aminocarboxyliques, d'hydrates et de sels de ceux-ci, en tant que substrats pour un système enzymatique couplé, comprenant une L-aminoacide désaminase ou oxydase et une déshydrogénase, pour la production par un traitement en lots d'acides α-hydroxycarboxyliques, d'hydrates et de sels de ceux-ci, dans lequel la concentration en substrat se situe entre 0,1 et 2 mol/litre.
